# EUROPEAN PATENT APPLICATION

(11) **EP 2 567 937 A1**
(43) Date of publication of application: **13.03.2013**
(21) Application number: 12004105.8
(22) Date of filing: 11.01.2008
(51) Int. Cl.: C01B 15/16, C01B 25/26, A61L 27/46, A61K 33/42

(54) **Biommetic hadroxyapatite composite materials and methods for the preparation thereof**

(30) Priority: 12.01.2007 US 622927
(62) Divisional of application: 08713737.8
(71) Applicant: Rutgers, The State University of New Jersey, New Brunswick, NJ 08903 (US)
(72) Inventor: Riman, Richard, NJ 08502 (US); Sever, Christina, NJ 08857 (US)
(74) Representative: Zacco GmbH

(57) **Abstract**

The present invention is related to methods for preparing composite materials, which include nanoscale hydroxyapatite, and the composite materials and articles prepared therewith.

## Description

### BACKGROUND OF THE INVENTION

Hydroxyapatite (HAp, chemical formula Ca₁₀(PO₄)₆(OH)₂) has attracted the attention of researchers over the past thirty years as an implant material because of its excellent biocompatibility and bioactivity. HAp has been extensively used in medicine for implant fabrication. It is commonly the material of choice for the fabrication of dense and porous bioceramics. Its general uses include biocompatible phase-reinforcement in composites, coatings on metal implants and granular fill for direct incorporation into human tissue. It has also been extensively investigated for non-medical applications such as a packing material/support for column chromatography, gas sensors and catalysts, as a host material for lasers, and as a plant growth substrate.

Previously explored methods of hydroxyapatite synthesis for particles include plasma spraying, hydrothermal synthesis, freeze drying, sol-gel, phase transformation, mechanochemical synthesis, chemical precipitation, and precipitation in simulated body fluid (SBF). All of these methods produce products with varying levels of purity, size, crystallinity, and yield. Plasma spraying, hydrothermal synthesis, sol-gel, phase transformation, mechanochemical synthesis, and chemical precipitation require elevated temperatures and/or extreme pH values in the fabrication of hydroxyapatite. These conditions can raise important questions among biologists when considering the material for in vivo applications because they are not biomimetic and, in most cases, do not yield biomimetic structures or morphologies. Furthermore, precipitation in simulated body fluid has such a low yield or long reaction time, it is not practical for use in manufacturing implants.

Therefore, a need exists for hydroxyapatite synthesis to take place at room temperature and optional neutral pH to allow the exploration of synthesis with live cells, including those in living organisms.

### SUMMARY OF THE INVENTION

There is provided, in accordance with the present invention, a method for preparing a composite material by (a) combining an amount of a calcium ion source, which includes calcium acetate, with a matrix material; (b) adding an amount of a phosphate ion source to the combination of step (a) to form a slurry having a pH from about 5.8 to about 14; and (c) removing water from the slurry of step (b) to produce the composite material, wherein the amounts of the calcium ion source and the phosphate ion source are sufficient to produce nanoscale hydroxyapatite under essentially ambient conditions.

Also provided is a method for preparing a composite material by (a) combining an amount of a calcium ion source, which includes calcium acetate, with an amount of a phosphate ion source to form a mixture having a pH from about 5.8 to about 14; (b) adding an amount of a solution, which includes citric acid and ammonium hydroxide, to the combination of step (a); (c) centrifuging the mixture of step (b) to form a supernatant and a precipitate, wherein the supernatant and the precipitate include hydroxyapatite particles; (d) combining a matrix material with the colloidal supernatant of step (c); and (e) removing water from the combination of step (d) to produce the composite material, wherein the amounts of the calcium ion source and the phosphate ion source are sufficient to produce nanoscale hydroxyapatite under essentially ambient conditions.

Also provided is a method for preparing a composite material by (a) combining an amount of a calcium ion source, which includes calcium acetate, with an amount of a phosphate ion source to form a mixture having a pH from about 5.8 to about 14; (b) adding an amount of a solution, which includes citric acid and ammonium hydroxide, to the combination of step (a); (c) centrifuging the mixture of step (b) to form a supernatant and a precipitate, wherein the supernatant and the precipitate include hydroxyapatite particles; (d) decanting the supernatant portion of step (c) from the precipitate portion; (e) allowing the precipitate portion of step (d) to form a colloidal gel; (f) combining a matrix material with the colloidal gel of step (e); and (g) removing water from the combination of step (f) to produce the composite material, wherein the amounts of the calcium ion source and the phosphate ion source are sufficient to produce nanoscale hydroxyapatite under essentially ambient conditions.

Also presented is a method for preparing a composite material by (a) combining an amount of a calcium ion source, which includes calcium acetate, with a matrix material; (b) injecting an amount of a phosphate ion source into the matrix material of step (a) to produce hydroxyapatite or a mixture of hydroxyapatite and a calcium phosphate at a pH from about 5.8 to about 14; (c) injecting an amount of the calcium ion source into the matrix material of step (b); and (d) optionally removing water from the matrix material of step (c), wherein the amounts of the calcium ion source and the phosphate ion source are sufficient to produce nanoscale hydroxyapatite under essentially ambient conditions.

Also provided is a method for preparing a composite material by (a) combining an amount of a calcium ion source, which includes calcium acetate, with a matrix material; (b) adding an amount of a phosphate ion source to the combination of step (a) to form a slurry having a pH from about 5.8 to about 14; and (c) pressing the slurry of step (b) to remove water from the slurry and produce the composite material, wherein the amounts of the calcium ion source and the phosphate ion source are sufficient to produce nanoscale hydroxyapatite under essentially ambient conditions.

Also provided is a composite material prepared according to a method of the present invention.

Also presented is a composite material, which includes hydroxyapatite particles and a matrix material, wherein the particles have a BET surface area between about 200 m²/g and about 3000 m²/g and a crystalline particle size between about 1nm and about 9nm.

Also presented is an article, which includes a composite material of the present invention.

Also provided is a kit for use in preparing a composite material, wherein the kit includes (a) an amount of a calcium ion source, which includes calcium acetate; (b) an amount of a phosphate ion source; and (c) a matrix material, wherein the amounts of the calcium ion source and the phosphate ion source are sufficient to produce nanoscale hydroxyapatite under essentially ambient conditions.

Also presented are powdered hydroxyapatite particles prepared by (a) obtaining an amount of a calcium ion source, which includes calcium acetate, (b) obtaining an amount of a phosphate ion source, and (c) combining the calcium ion source and the phosphate ion source, wherein the amounts of the calcium ion source and the phosphate ion source are sufficient to produce nanoscale hydroxyapatite particles and the amounts are combined under essentially ambient conditions to produce the hydroxyapatite particles; wherein one or more dopant ions suitable for substitution into the HAp lattice, one or more sintering or processing additives, a pharmaceutically active composition, or a combination thereof are added to any of steps (a) - (c).

### BRIEF DESCRIPTION OF THE DRAWINGS

FIGS. 1a-d are micro computed tomography (µ-CT) scans of a mineralized intact fiber matrix;
FIGS. 2a-b are transmission electron microscopy (TEM) images of the mineralized intact fiber matrix;
FIGS. 3a-b are µ-CT scans of a mineralized bone powder;
FIG. 4 is a µ-CT scan of a mineralized PLGA polymer explant following 4 weeks of implantation in a femoral defect of a rabbit;
FIGS. 5a-b are x-ray diffraction (XRD) spectra corresponding to compositions prepared according to the method of Example 9; and
FIG. 6 is an x-ray diffraction (XRD) spectrum corresponding to a composition prepared according to the method of Example 10.

### DETAILED DESCRIPTION OF THE INVENTION

The present invention is related to methods for preparing composite materials, which include nanoscale hydroxyapatite, and the composite materials and articles prepared therewith.

Hydroxyapatite has reported uses for biomedical, chromatographic, and piezoelectric applications and has been synthesized by various techniques. However, reaction conditions for the preparation of HAp such as high temperatures, high pressures and extreme pH values, as well as low yield, vigorous washing requirements, and long reaction times limit biological applications.

The methods of the present invention permit the formation under mild reaction conditions of HAp under conditions suitable for the above uses, especially biological use. The methods of the present invention include dynamic and static methods for introducing hydroxyapatite onto a matrix material. "Static" refers to depositing pre-made hydroxyapatite particles on a matrix material. "Dynamic" refers to the formation of hydroxyapatite on the matrix material by depositing calcium ions onto the matrix material followed by subsequent reaction with phosphate ions to produce hydroxyapatite.

One method involves (a) combining an amount of a calcium ion source, which includes calcium acetate with a matrix material; (b) adding an amount of a phosphate ion source to the combination of step (a) to form a slurry having a pH from about 5.8 to about 14; and (c) removing water from the slurry of step (b) to produce the composite material, wherein the amounts of the calcium ion source and the phosphate ion source are sufficient to produce nanoscale hydroxyapatite under essentially ambient conditions.

In one embodiment, the slurry is introduced into a mold prior to step (c). In another embodiment, the slurry is introduced into a colloid press prior to step (c).

Another method involves (a) combining an amount of a calcium ion source, which includes calcium acetate, with an amount of a phosphate ion source to form a mixture having a pH from about 5.8 to about 14; (b) adding an amount of a solution, which includes citric acid and ammonium hydroxide, to the combination of step (a); (c) centrifuging the mixture of step (b) to form a supernatant and a precipitate, wherein the supernatant and the precipitate include hydroxyapatite particles; (d) combining a matrix material with the colloidal supernatant of step (c); and (e) removing water from the combination of step (d) to produce the composite material, wherein the amounts of the calcium ion source and said phosphate ion source are sufficient to produce nanoscale hydroxyapatite under essentially ambient conditions.

Yet another method for preparing a composite material includes (a)combining an amount of a calcium ion source, which includes calcium acetate, with an amount of a phosphate ion source to form a mixture having a pH from about 5.8 to about 14; (b) adding an amount of a solution comprising citric acid and ammonium hydroxide to the combination of step (a); (c) centrifuging the mixture of step (b) to form a supernatant and a precipitate, wherein the supernatant and the precipitate include hydroxyapatite particles; (d) decanting the supernatant portion of step (c) from the precipitate portion; (e) allowing the precipitate portion of step (d) to form a colloidal gel; (f) combining a matrix material with the colloidal gel of step (e); and (g) removing water from the combination of step (f) to produce the composite material, wherein the amounts of the calcium ion source and the phosphate ion source are sufficient to produce nanoscale hydroxyapatite under essentially ambient conditions.

Another method includes (a) combining an amount of a calcium ion source, which includes calcium acetate, with a matrix material; (b) injecting an amount of a phosphate ion source into the matrix material of step (a) to produce hydroxyapatite or a mixture of hydroxyapatite and a calcium phosphate at a pH from about 5.8 to about 14; (c) injecting an amount of the calcium ion source into the matrix material of step (b); and (d) optionally removing water from the matrix material of step (c), wherein the amounts of the calcium ion source and the phosphate ion source are sufficient to produce nanoscale hydroxyapatite under essentially ambient conditions.

In one embodiment, the calcium phosphate is selected from monetite, brushite, calcite, tricalcium phosphate, whitlockite, and combinations thereof.

In another embodiment, step (a) includes soaking the matrix material in a solution of the calcium ion source. In an additional embodiment, the matrix material is soaked for about 1 minute to about 48 hours.

Yet another method includes (a) combining an amount of a calcium ion source, which includes calcium acetate, with a matrix material; (b) adding an amount of a phosphate ion source to the combination of step (a) to form a slurry having a pH from about 5.8 to about 14; and (c) pressing the slurry of step (b) to remove water from the slurry and produce the composite material, wherein the amounts of the calcium ion source and the phosphate ion source are sufficient to produce nanoscale hydroxyapatite under essentially ambient conditions. Preferably, step (c) is performed with a colloidal press or a filter press. In general, a colloidal press is designed to densify and remove water (or aqueous solution) from a colloidal system, while impeding the loss of particles during pressing or processing.

The pH range mentioned in the methods discussed above is from about 5.8 to about 14. In another embodiment, the pH range is from about 5.8 to about 8.5.

When the calcium ion source is in solution, a preferred ion concentration is from about 0.01 millimolal to about 2.0 molal. When the phosphate ion source is in solution, a preferred ion concentration is from about 0.006 millimolal to about 1.2 molal. If a particular ion source is not in solution, the source is in a solid phase.

Optionally, the phosphate ion source, or a portion thereof, is neutralized (e.g. pH adjusted to ∼ 7.4) prior to combining with the calcium ion source. This step allows the slurry to form more quickly.

Suitable phosphate ion sources include, but are not limited to, one or more of potassium or sodium orthophosphate; orthophosphoric acid; Group I phosphates, preferably monobasic, dibasic, or tribasic potassium or sodium phosphate; magnesium phosphate; ammonium phosphate; ammonium phosphate tribasic; and the like. Potassium or sodium orthophosphate is preferred. In addition to calcium acetate, the calcium ion source may also include one or more of calcium hydroxide, calcium oxalate, calcium nitrate, calcium phosphate, calcium carbonate, calcium citrate, calcium fluoride, and calcium chloride. Calcium acetate alone is preferred.

The calcium ion source, the phosphate ion source, or both are in solution prior to combining the sources. Preferably, the solution contains one or more of water, buffer, solvent, simulated body fluid, or fortified cell medium with or without serum. Suitable buffers include, but are not limited to, N-(2-hydroxyethyl)-piperazine-N'-2-ethanesulfonic acid (HEPES), 2-(bis(2-hydroxyethyl)amino)-2-(hydroxymethyl)propane-1,3-diol (BIS-TRIS), 3-(N-Morpholino)-propanesulfonic acid (MOPS), N-(2-Acetamido)-2-aminoethanesulfonic acid (ACES), N-(2-Acetamido)iminodiacetic Acid (ADA), N,N-Bis(2-hydroxyethyl)-2-aminoethanesulfonic Acid (BES), 3-[N,N-bis(2-hydroxyethyl)amino]-2-hydroxypropanesulfonic acid (DIPSO), 4-(N-morpholino)butanesulfonic acid (MOBS), 3-[N-morpholino]-2-hydroxypropanesulfonic acid (MOPSO), piperazine-1,4-bis(2-ethanesulfonic acid) (PIPES), 3-[N-Tris(hydroxymethyl)methylamino]-2-hydroxypropanesulfonic acid (TAPSO), N-Tris(hydroxymethyl)methyl-2-aminoethanesulfonic acid (TES), and acetic acid. A preferred buffer is acetic acid.

Matrix materials suitable for use in preparing the composite materials of the present invention include those for which an osteoconductive coating is desired. Exemplary matrix materials include demineralized bone (e.g. Grafton® DBM, Osteotech, Inc., Eatontown, New Jersey), mineralized bone (e.g. Plexur™, Osteotech, Inc., Eatontown, New Jersey), collagen, silks, polymeric materials, and combinations thereof. Preferred matrices include those which are osteoinductive and/or osteoconductive. The matrix material can have any suitable shape or form for implantation in the body of a patient in need thereof. Exemplary shapes and forms include fibers (e.g. Grafton® DBM Orthoblend), fiber mats (e.g. Grafton® DBM Matrix PLF), cubes, cylindrical forms (e.g. Grafton® DBM Matrix Plugs), flexible forms (e.g. Grafton® DBM Flex), putties (e.g. Grafton® DBM Putty), gels (e.g. Grafton® DBM Gel), pastes (e.g. Grafton® DBM Paste), strips (e.g. Grafton® DBM Matrix Strips), powders, chips, and combinations thereof (e.g Grafton® DBM Crunch).

In one embodiment, the composite material includes nanoscale hydroxyapatite distributed throughout the matrix, a matrix material (e.g. demineralized bone, mineralized bone, collagen, silks, polymeric materials, and combinations thereof) having at least a portion coated with nanoscale hydroxyapatite, or combinations thereof. For example, nanoscale hydroxyapatite can be distributed throughout an individual matrix powder particle or a matrix powder particle can be coated with nanoscale hydroxyapatite. In one embodiment, a calcium affinity additive is added to the matrix material prior to the formation of hydroxyapatite to increase bonding between the hydroxyapatite and the matrix material. Exemplary calcium affinity additives include, but are not limited to, troponin C, calmodulin, calcitriol, ergocalciferol, serum albumin, chitin, phosphophoryn, elastin, and fibrin.

In another embodiment the composite material is incorporated into an osseous cement. For example, a composite material having a powder particle matrix can be incorporated into an osseous cement.

In one embodiment, the polymeric matrix material is soaked in ethanol (pH ∼ 7) prior to preparing the hydroxyapatite coating. This treatment step decreases the surface tension of the polymeric material, which enhances the penetrability of porous polymeric materials.

Suitable polymers include polysaccharides, poly(alkylene oxides), polyarylates, for example those disclosed in U.S. Patent No. 5,216,115, block copolymers of poly(alkylene oxides) with polycarbonates, for example those disclosed in U.S. Patent No. 5,658,995, polycarbonates, for example those disclosed in U.S. Patent No. 5,670,602, free acid polycarbonates, for example those disclosed in U.S. Patent No. 6,120,491, polyamide carbonates and polyester amides of hydroxy acids, for example those disclosed in U.S. Patent No. 6,284,862, polymers of L-tyrosine derived diphenol compounds, including polythiocarbonates and polyethers, for example those disclosed in U.S. Patent No. RE37,795, strictly alternating poly(alkylene oxide) ethers, for example those disclosed in U.S. Patent No. 6,602,497, polymers listed on the United States FDA "EAFUS" list, including polyacrylamide, polyacrylamide resin, modified poly(acrylic acid-co-hypophosphite), sodium salt polyacrylic acid, sodium salt poly(alkyl(C16-22) acrylate), polydextrose, poly(divinylbenzene-co-ethylstyrene), poly(divinylbenzene-co-trimethyl(vinylbenzyl)ammonium chloride), polyethylene (m.w. 2,00-21,000), polyethylene glycol, polyethylene glycol (400) dioleate, polyethylene (oxidized), polyethyleneimine reaction product with 1,2-dichloroethane, polyglycerol esters of fatty acids, polyglyceryl phthalate ester of coconut oil fatty acids, polyisobutylene (min. m.w. 37,000), polylimonene, polymaleic acid, polymaleic acid, sodium salt, poly(maleic anhydride), sodium salt, polyoxyethylene dioleate, polyoxyethylene (600) dioleate, polyoxyethylene (600) mono-rici noleate, polyoxyethylene 40 monostearate, polypropylene glycol (m.w. 1,200-3,000), polysorbate 20, polysorbate 60, polysorbate 65, polysorbate 80, polystyrene, cross-linked, chloromethylated, then aminated with trimethylamine, dimethylamine, diethylenetriamine, or triethanolamine, polyvinyl acetate, polyvinyl alcohol, polyvinyl polypyrrolidone, and polyvinylpyrrolidone, and polymers listed in U.S. Patent No. 7,112,417, the disclosures of all of which are incorporated herein by reference in their entirety, Preferred polymers include: polyamides, polyesters (e.g. Dacron®), polycaprolactone (PCL), polyglycolide-co-caprolactone, polyethylene oxide (PEO), polypropylene oxide (PPO), polyglycolide-co-trimethylene carbonate (PGA-co-TMC), poly(lactic-co-glycolic acid) (PLGA), polylactide (PLA), polyglycolic acid (PGA), poly-L-lactide (PLLA), polyethylene glycol (PEG), polypropylene (PP), polyethylene (PE), and polyetheretherketones (PEEK).

An optional step includes agitating the calcium ion source/phosphate ion source/matrix combination until HAp is formed. Agitating the combination accelerates the formation of hydroxyapatite. As used herein, the term "agitate" refers to mechanical movement, for example, vibrating, vortexing, swirling, shaking, ultrasonicating, stirring, or the like that causes mixing. Mechanical movements include movements performed by hand.

Essentially ambient conditions are employed. A preferred temperature range is between -10°C and 45°C. At room temperature, HAp is typically produced within 1 minute to an hour. Combining the sources while beating will speed up the rate of reaction to more quickly produce HAp, while combining the ion sources while cooling will decrease the rate at which HAp forms.

During the course of the reaction, a pH swing may occur, which is varied with the calcium to phosphate stoichiometry.

The employment of a buffer as the reaction medium moderates the pH change, which affects the product formed. Hydroxyapatite is formed, but secondary phases of calcium phosphate and calcium carbonate may be additionally formed, but can be remedied through process variations, for example, bubbling with nitrogen, addition of chelating agents, or use of additional pH adjustments or buffers.

An optional washing step can be performed following the combination of the calcium ion source and the phosphate ion source. This step includes, for example, filtration, centrifuging, and/or liquid replacement. Centrifuging or liquid replacement are preferred. Minimal washing cycles are needed because of the non-toxic nature of the ions left in solution. In one embodiment, the citrate wash disclosed in U.S. Patent No. 6,921,544, the contents of which are incorporated herein by reference in their entirety, is used to remove at least a portion of an amorphous phase if the amorphous phase is considered an undesired impurity. In another embodiment, the hydroxyapatite is washed with a buffer solution.

Another optional step includes adding a pharmaceutically active composition or one or more dopant ions suitable for substitution into the HAp lattice. Preferably, the dopant ions and/or pharmaceutically active composition dopant are added to the calcium ion source, the phosphate ion source, or a combination of the sources. Dopant ions are readily determinable by one of skill in the art. Suitable ions include, but are not limited to, magnesium, fluorine, chlorine, potassium, iron, carbonate, sodium, barium, strontium, and the like. The HAp particles of the present invention can also be doped with ions of one or more rare earth elements. Suitable pharmaceutically active compositions include those mentioned below.

Yet another optional step includes introducing one or more additives selected from pharmaceutically active compositions, proteins, polymer precursor compositions, polymers, biomarkers (e.g. ligands, radioisotopes, etc.), and combinations thereof in a step prior to the water removal step. For example, proteins, polymer precursor compositions, polymers, or combinations thereof can be included with the calcium ion source prior to its combination with the phosphate ion source.

Another optional step includes introducing one or more additives selected from proteins, polymers, and combinations thereof to the composite material.

Additional additives include sintering and processing additives, for example, CaO, P₂O₅, Na₂O, MgO, and the like.

Proteins can enhance osteoconductivity and osteoinductivity of the composite materials. Exemplary proteins include osteocalcin, osteonectin, bone morphogenetic proteins (BMPs), interleukins (ILs), glycosaminoglycans, proteoglycans, growth factors, fibrin, fibrinogen, chitosan, osteoinductive factor, fibronectin, human growth hormone, insulin-like growth factor, soft tissue, bone marrow, serum, blood, bioadhesives, human alpha thrombin, transforming growth factor beta, epidermal growth factor, platelet-derived growth factors, fibroglast growth factors, periodontal ligament chemotactic factor, somatotropin, bone digestors, antitumor agents, immuno-suppresants, permeation enhancers, enamine derivatives, alpha-keto aldehydes, nucleic acids, amino acids, and gelatin.

Polymeric additives enhance the strength and/or osteoconductlvity of the composite material. Exemplary polymers include those mentioned above.

To produce solid hydroxyapatite, the calcium ion source/phosphate ion soume/matrix combination is dried. Suitable drying techniques are readily determinable by those of skill in the art. Preferred drying techniques include evaporative and sublimation-based drying methods, for example, oven drying and freeze drying. The composite material can also be dried in a desiccator.

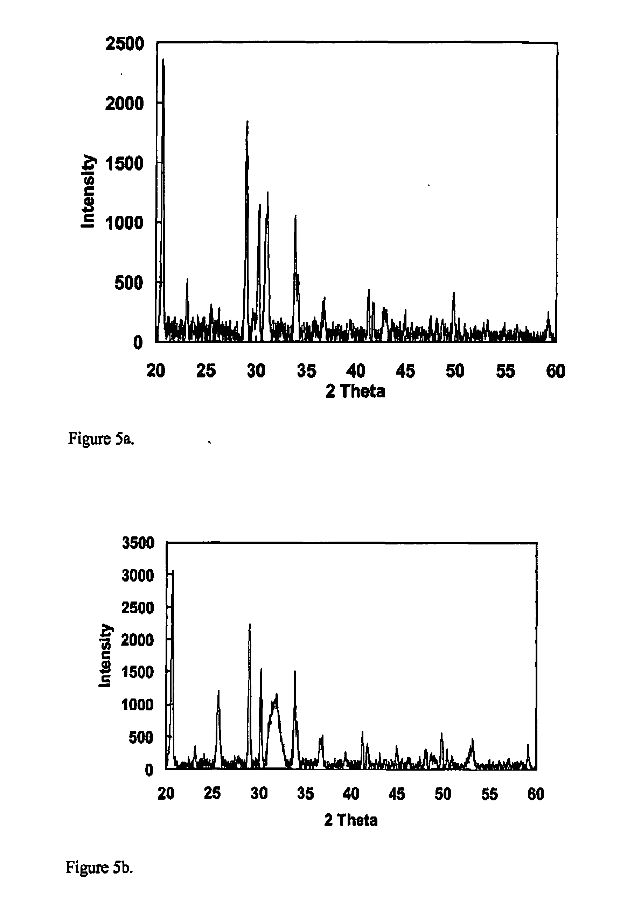

The composition of the HAp formed on the composite material is stoichiometric or non-stoichiometric with respect to calcium and phosphate. For example, the XRD diffraction pattern of FIG. 5(b) represents the results of a standard test for stoichiometry for a cast sample. This figure shows the presence of peaks corresponding to monetite (CaHPO₄), hydroxyapatite, and potassium calcium phosphate after the sample was heat treated at 900°C for 2 hours. The presence of monetite or other calcium phosphates indicates a non-stoichiometric composition and/or an amorphous phase. In one embodiment, at least a portion of the composition includes an amorphous phase.

The methods according to the present invention can taka place in any suitable reaction system.

An optional technique for combining the calcium ion source, phosphate ion source, and matrix material is electrospinning. For example, the calcium ion source and a polymer precursor solution are combined in one syringe pump. The phosphate ion source and a solvent are combined in another syringe pump. The contents of the syringes are discharged and mixed in a mixing chamber just prior to being formed into an ultrafine fiber through the application of high voltage and evaporation of the solvent. The fiber can be used to form a fibrous mat, which can be further functionalized with the protein and polymeric additives discussed herein.

Another optional technique for combining the calcium ion source, phosphate ion source, and matrix material is spray deposition, wherein the ion sources are deposited on the surface of the matrix material.

Given that hydroxyapatite has no toxicity and its components are low cost, such a technology presents great promise for a range of applications. For example, composite materials of the present invention did not dissociate while submerged in water for an extended period of time, which makes them useful as bone implant materials.

Therefore, another embodiment includes a composite material prepared according to any method of the present invention.

Also presented is a composite material, which includes hydroxyapatite particles and a matrix material, wherein the particles have a BET surface area between about 200m²/g and about 3000m²/g and a crystalline particle size between about 1nm and about 9nm. Particle size is calculated from surface area measurements via the BET method with the equation: Particle size = shape factor/(surface area*density of the particles). The shape factor is assumed as 1 (for spherical particles) and the density has been measured as 2.5g/cm³ with helium pycnometry.

Preferably, the composite material includes a total amount of calcium phosphate mineral from about 0.01% to about 50% by weight of the composite material. A lower mineral content is preferred when retention of osteoinductive protein viability is desired. Higher mineral contents are preferred for structural and strengthening purposes.

The matrix material can have any suitable shape or form for implantation in the body of a patient in need thereof. Exemplary shapes and forms are mentioned above.

In one embodiment, the ion ratio of calcium to phosphate in the composite material is between 1.25 and 4 In another embodiment, the hydroxyapatite particles are doped with a pharmaceutically active composition or one or more ions suitable for substitution into the HAp lattice.

Optionally, the composite material includes one or more additives selected from pharmaceutically active compositions, proteins, polymers, and combinations thereof. Exemplary proteins and polymers are mentioned above.

In one embodiment, the composite material includes stoichiometric or non-stoichiometric hydroxyapatite.

Also presented are articles incorporating any of the composite materials of the present invention. Preferred articles include, for example, intervertebral dowels, intervertebral spacers, intervertebral implants, osteogenic bands, osteoimplants, bone implants, bone powders, bone particles, bone grafts, shaped demineralized bone, demineralized bone powders, mineralized bone powders, hip stems, dental implants, and shaped osteoimplants.

Optionally, the article includes a pharmaceutically active composition. Preferred pharmaceutically active compositions include compositions for treating bone disease (e.g. bisphosphonates, alendronate, strontium ranelate, teriparatide, etc.), compositions for preventing bone loss (e.g. steroids, for example, Estradiol Cypionate, Ethynyl Estradiol, Mestranol, Quinestrol, Exemestane, Testolactone, Norethindrone, Norethynodrel, Levonorgestrel, mifepristone, etc.) and compositions for treating cancer (e.g. alkylating agents, antimetabolites, anthracyclines, alkaloids, topoisomerase inhibitors, monoclonal antibodies, tyrosine kinase inhibitors, antitumor antibiotics, paclitaxel, platinating agents such as Cisplatin, Carboplatin, Oxaliplatin. Mechlorethamine, Chlorambucil, Cyclophosphamide, Ifosfamide, Busulfan, Camustine, Dacrbazine, Temozolomide, Procarbazine hydrochloride, Thiotepa, 5-Fluorouracil, Floxuridine, Capecitabine, Gemcitabine, Cytarabine, 6-mercaptopurine, 6-thioguanine, Fludarabine phosphate, Cladribine, Clofaxabine, Pentostatin, Methotrexate, paclitaxel, Docetaxel, Vincristine, Viblastine, Vinorelbine, camptothecin, Irinotecan, Topotecan, 5H-Dibenzo[c,h][1,6]-naphthyrindi-6-ones ARC-111, Etoposide, Doxorubicin, Daunorubicin, Idarubicin, Novatrone, Bleomycin, Dactinomycin, Mitomycin, hydroxyurea, L-Asparaginase, Estramustine, Imatinib Mesylate, Dasatinib, Sorafenib, Sunitinib, Amifostine, MESNA, Dexrazoxane, Lucovorin Calcium, steroids, and antiestrogens (e.g. Tarnoxifen citrate, Toremifene citrate, Enclomiphene citrate, Zuclomiphene, Anastrozale, Letrozole, etc.)). Suitable pharmaceutically active compositions also include those mentioned below.

Also presented is a kit for use in preparing composite materials of the present invention. The kit includes (a) an amount of a calcium ion source comprising calcium acetate; (b) an amount of a phosphate ion source; and (c) a matrix material, wherein the amounts of the calcium ion source and the phosphate ion source are sufficient to produce nanoscale hydroxyapatite under essentially ambient conditions. The two ion sources are provided in separate containers. Other components may be present depending upon the intended therapeutic use.

Also presented are powdered hydroxyapatite particles prepared by combining an amount of a calcium ion source, which includes calcium acetate, and an amount of a phosphate ion source, wherein the amounts are sufficient to produce nanoscale HAp particles and the amounts are combined under essentially ambient conditions to produce the HAp particles.

In one embodiment, the powdered hydroxyapatite particles encapsulate or are at least partially coated with therapeutic cells (e.g. stem cells). These particles can be further included in a composite material or an article of the present invention.

In another embodiment, the powdered hydroxyapatite particles further include a biomarker (e.g. ligand, radioisotope, etc.)

Also presented are powdered hydroxyapatite particles prepared by (a) obtaining an amount of a calcium ion source, which includes calcium acetate, (b) obtaining an amount of a phosphate ion source, and (c) combining the calcium ion source and the phosphate ion source, wherein the amounts of the calcium ion source and the phosphate ion source are sufficient to produce nanoscale hydroxyapatite particles and the amounts are combined under essentially ambient conditions to produce the hydroxyapatite particles; wherein one or more dopant ions suitable for substitution into the HAp lattice, one or more sintering or processing additives, a pharmaceutically active composition, or a combination thereof are added to any of steps (a) - (c). Preferred sintering or processing additives include CaO, P₂O₅, Na₂O, MgO, and the like.

In one embodiment, the powdered hydroxyapatite particles are sintered.

Suitable dopant ions for the powdered hydroxyapatite particles are readily determinable by one of skill in the art. Suitable ions include, but are not limited to, magnesium, fluorine, chlorine, potassium, iron, carbonate, sodium, barium, strontium, chromium, vanadium, elements of the lanthanide series (e.g. ytterbium, erbium, neodymium, and thulium), Group 13 elements suitable for use es p-type dopants (e.g. boron, aluminum, gallium, indium, and thalium), Group 15 elements suitable for use as n-type dopants (e.g. nitrogen, phosphorous, arsenic, antimony, and bismuth), and the like. The HAp particles of the present invention can also be doped with ions of one or more rare earth elements.

Depending upon the presence of particular dopant(s) and/or additive(s), exemplary uses for the hydroxyapatite particles include; solid-state laser media, semiconductors, x-ray contrast materials, paint pigments, household cleaners, rubber additives, sealant additives, fertilizers, conductive materials, paper processing, calcium nutritional supplements, food additives (e.g. anticaking agents), drug delivery, cosmetics (e.g. powder foundation, liquid foundation, lipstick, eyeshadow, blush, liners, pencils, bronzers, and the like), and toothpaste.

Undoped powdered hydroxyapatite particles prepared by combining an amount of a calcium ion source, which includes calcium acetate, and an amount of a phosphate ion source, wherein the amounts are sufficient to produce nanoscale HAp particles and the amounts are combined under essentially ambient conditions to produce the HAp particles can also be utilized as mentioned above. Preferred uses for undoped powdered hydroxyapatite particles include: radioopaque imaging agents, paint pigments, household cleaners, rubber additives, sealant additives, fertilizers, paper processing, calcium nutritional supplements, food additives (e.g. anticaking agents), cosmetics (e.g. powder foundation, liquid foundation, lipstick, eyeshadow, blush, liners, pencils, bronzers, and the like), toothpaste, and drug delivery (e.g. oral tableting and intravenous infusion).

Hydroxyapatite particles having the size distribution of the present invention (e.g. a BET surface area between about 200 and about 3000 m²/g and a particle size between about 1 nm and about 9 nm) are effective in drug delivery because they are more capable of penetrating the cellular wall and carry a much higher surface area for adsorption of drug molecules. The range also allows the particles to be used intravenously as a drug therapy, for transdermal drug delivery, or for oral tableting.

Suitable pharmaceutically active compositions for incorporation into the hydroxyapatite particles, in addition to the compositions mentioned above, include antibiotics, pain relievers, analgesics, nutritional supplements, antihistamines, NSAIDS, antipsyohotics, antichoinergics, cholinergics, antisposmotics, adrenergic agonists and antagonists (alpha and beta blockers), antidepressants, diabetes treatments, antivirals, dopaminergic agents, seratonergic agents, PDEIs (phosphodiesterase inhibitors), cardiac stimulants, suppressants, gastrointestinal drugs, antilipidemics, antihypertensive agents, diuretics, enzyme inhibitors, ion channel blockers, antifungal agents, steroids, blood glucose regulators, antiepileptics, anesthetics, skeletal muscle relaxants, prostaglandins, sedatives, analeptics, antineoplastics (antitumor), antiprotozoals, antihelminthics, hypnotics, antiemetics, antianginal, antiarrhythmics, vasodilators, vasocontrictors, antiulcer agents, antiallergics, antacids, gene transfection, and the like.

The following non-limiting examples set forth herein below illustrate certain aspects of the invention.

### EXAMPLES

### Example 1 - Solution preparation.

Calcium acetate hydrate (99% Acros Organics, Belgium, CAS # 114460-21-8) and potassium orthophosphate hydrate (Acros Organics, Belgium, CAS# 27176-10-9) were used as reactants for the synthesis of hydroxyapatite. First, a 1.0 molal calcium acetate hydrate solution was made using distilled, deionized water ("calcium solution"). Then, a 0.6 molal solution of potassium orthophosphate hydrate was made using distilled, deionized water. The solution was divided in half ("phosphate solutions") and acetic acid was added to one solution until the pH reached 7.4 ("neutralized solution"). The volume of acetic acid depends on total solution volume. For example, a 500mL solution needs about 23mL of glacial acetic acid.

### Example 2 - Surface mineralization of an intact fiber matrix.

Demineralized bone matrix (0.7416 g) in the form of a fiber mat (Grafton Matrix, Osteotech, Inc., Eatontown, NJ) was soaked in 10mL of the calcium solution until hydrated (about 1 hour). Phosphate solutions were added as follows: 8.5mL of the un-neutralized solution was added, followed by 1.5mL of the neutralized solution. All 4 components were then covered and vortexed until a thin white slurry results (about 2 minutes). The fiber mat was then extracted and washed in distilled, deionized water 3 times or until the resulting solution remained clear when agitated. This action should dislodge any hydroxyapatite not precipitated on the surface. The mat was then put in a 45 °C oven for a period of about 3 hours, then frozen and lyophilized.

Samples for XRD were prepared by drying residual powder from the washes and placing the powder on amorphous double sided tape. The samples were then introduced into the diffractometer. Angles from 20-80 degrees were scanned using 0.3 step size and 3 second dwell time. CuK_{α} source was used in the Siemens Krystalloflex Diffractometer.

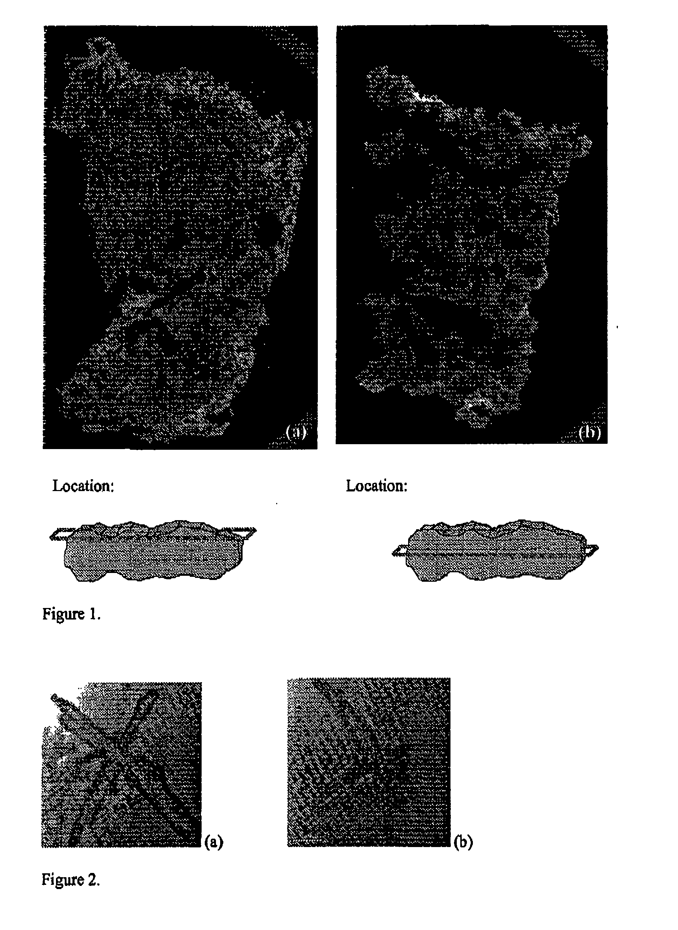

Samples for µ-CT are directly placed in the instrument. This method of analysis is effective in determining relative mineral content in a sample being that a sample with no mineral content will show a blank picture and a sample with a high content of mineral will show stark white. FIG. 1 shows mineralization of the fiber matrix.

Samples for TEM are prepared by preparing low viscosity Spurr's epoxy and embedding 0,05 gram of mineralized fibers in the point of the TEM mold. The embedded sample is then ultramicrotomed to 70nm sections and placed on TEM grids for analysis. FIGS. 2(a) and 2(b) show full mineralization of the surface of the macroscopic fiber surface.

The presence of calcium was confirmed by staining the mineralized polymer with Alizarin red. Alizarin red binds calcium in a semi-quantitative way, a more uniform darker red color indicates a high amount of calcium. These samples had a uniform dark red color. Semi-quantitative analysis can only be done when all samples have been subjected to stain of the same amount for the same amount of time from the same batch.

### Example 3 - Surface mineralization of a dissociated fiber matrix.

Demineralized bone matrix (0.7416 g) (Grafton Matrix, Osteotech, Inc., Eatontown, NJ) was soaked in 10mL of the calcium solution until hydrated. The matrix was then broken apart to create a fibrous slurry. Phosphate solutions were added as follows: 8,5mL of the un-neutralized solution was added, followed by 1.5mL of the neutralized solution. All 4 components were then covered and vortexed until a thin white slurry results (about 2 minutes). The entire solution was extracted and centrifuged for 5 minutes at 3000 rpm, 4G. The remaining liquid was poured off. The mineralized fibers were then transferred to a mold and dried in a 45 °C oven for 3 hours, then frozen and lyophilized.

### Example 4 - Demineralized Powder Mineralization.

Demineralized bone powder (0.7416 g) (Grafton Gel without Glycerol, Osteotech, Inc., Eatontown, NJ) was soaked in 3mL of the calcium solution for 24 hours. Phosphate solutions were added as follows: 2.8mL of the un-neutralized solution was added, followed by 0.2mL of the neutralized solution. All 4 components were then stirred for 2 minutes passing the viscous stage. The resulting slurry was then dried overnight in a 45 °C oven (for a spongy compact) or washed thoroughly with distilled, deionized water on a fine sieve and dried.

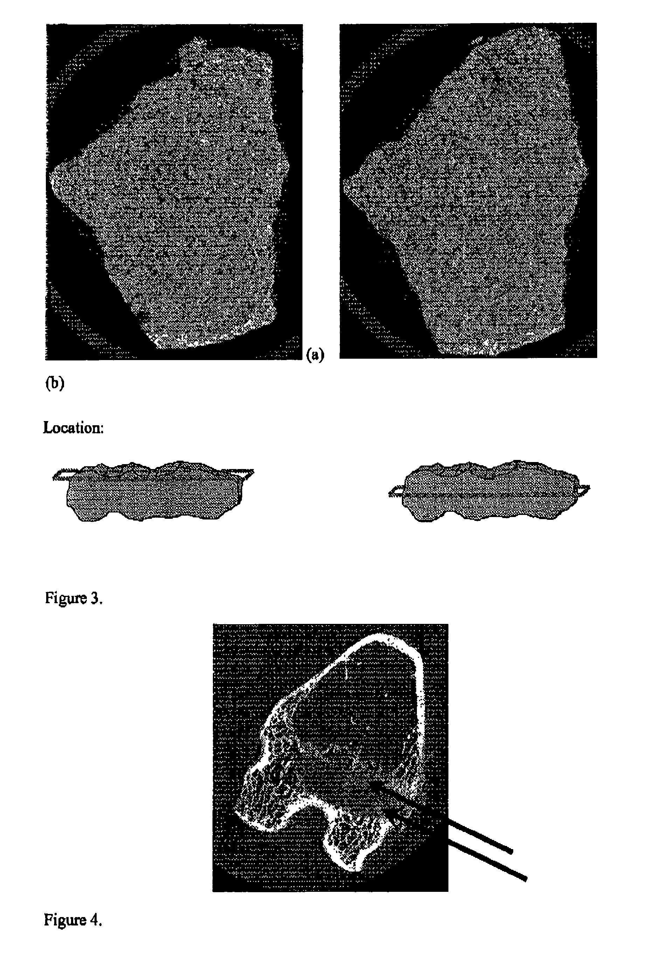

Mineralization of the powder was confirmed by micro-CT. (FIG. 3).

### Example 5 - Mineralization of a porous PLGA polymer.

Porous PLGA polymer (0,7416g) was soaked briefly in fresh pH 6 ethanol then in 10mL of the calcium solution until hydrated (about 1 hour to 24 hours). Phosphate solutions were added as follows: 8.5mL of the unneutralized solution was added, followed by 1.5mL of the neutralized solution. All 4 components were then covered and vortexed until a thin white slurry resulted (about 2 minutes). The polymer was then extracted and washed in distilled, deionized water 3 times or until the resulting solution remained clear when agitated. This action should dislodge any hydroxyapatite not precipitated on the surface. The polymer is then used directly or put in a 35 °C oven for 4 hours. The presence of calcium was confirmed by staining the mineralized polymer with Alizarin red.

A mineralized PLGA polymer was surgically implanted in a femoral defect of a rabbit. After 4 weeks, the implant was removed. Bone formation (arrows) was observed at the implant site. (FIG. 4).

### Example 6 - Light mineralization of fibers via colloidal suspension soak.

Calcium acetate hydrate (99% Acros Organics, Belgium, CAS # 114460-21-8) and potassium orthophosphate hydrate (Acros Organics, Belgium, CAS# 27176-10-9) were used as reactants for the synthesis of colloidal hydroxyapatite. First, a 1.0 molal calcium acetate hydrate solution was made using distilled, deionized water. Then, a 0.6 molal solution of potassium orthophosphate hydrate was made using distilled, deionized water. A citric acid wash was made by making a 0.2M solution of citric acid and adding ammonium hydroxide until pH=8.9.

100mL of the calcium solution and 100mL of the phosphate solution were mixed and stirred thoroughly through the viscous gel-like stage. Following this step, 1000mL of the 0.2M citric acid wash was added and allowed to stir overnight or for at least 12 hours. This mixture was then centrifuged at 4000rpm for 6 minutes. The colloidal supernatant (remaining liquid with unsettled particles dispersed, now a colloidal suspension) was saved and considered a suspension of the smallest particles precipitated in the reaction, Five grams of demineralized fibers or fiber mat was then soaked in the colloidal supernatant for 24 hours, removed, and dried in a 45 °C oven overnight. The presence of calcium was confirmed by staining the mineralized fibers with Alizarin red.

### Example 7 - Preparation of colloidal gel,

The supernatant is decanted from the centrifuged mixture prepared according to Example 6. The centrifuge tube containing the precipitate is covered and allowed to sit for 3 days. After 3 days, a colloidal gel is observed. Upon agitation, the gel becomes a lower viscosity liquid.

### Example 8 - Colloidal pressing of fibers.

Calcium acetate hydrate (99% Acros Organics, Belgium, CAS # 114460-21.8) and potassium orthophosphate hydrate (Acros Organics, Belgium, CAS# 27176-10-9) are used as reactants for the synthesis of hydroxyapatite. First, a 1.0 molal calcium acetate hydrate solution is made using distilled, deionized water. Then, a 0.6 molal solution of potassium orthophosphate hydrate is made using distilled, deionized water. The phosphate solution is divided in half and acetic acid is added to one solution until the pH reaches 7.4 (neutralized solution). The volume of acetic aicd depends on total solution volume. For example, a 500mL solution needs about 23mL of glacial acetic acid.

Demineralized bone matrix (10g) (Grafton Matrix, Osteotech, Inc., Eatontown, NJ) is soaked in 100mL of the calcium solution until hydrated (about 1 hour). Phosphate solutions are added as follows: 85mL of the unneutralized solution is added, followed by 15mL of the neutralized solution. All 4 components are then stirred until a thin white slurry results.

The mixture is then put into a colloidal press and pressed with a Carver press to draw out the liquid reaction medium, leaving the mineralized fibers and remaining mineral to be pressed into a strong cohesive pellet. The pellet is then put in a 45 °C oven overnight to remove any residual moisture.

### Example 9 - Injection mineralization of an intact fiber matrix.

Demineralized bone matrix (0.7416g) (Grafton Matrix, Osteotech, Inc., Eatontown, NJ) was soaked in 10mL of the calcium solution until hydrated (about 1 hour). The matrix was then placed on top of a 0.2m PES membrane nalgene filter and a vacuum was pulled to remove excess liquid from the matrix. While the matrix was still on the filter, a 22-gage needle on a syringe was filled with the phosphate solution and an identical one filled with the calcium solution. About 5 mL total of phosphate solution was injected at 15 sites in the matrix while the vacuum pump was on. This step was repeated with the calcium solution, followed by the phosphate solution. The alternating calcium and phosphate solutions were injected as such until the matrix no longer accepted the needle due to a high mineral content. The matrix was then flipped over and the process was repeated on the opposite side.

Samples for XRD were prepared by taking residual powder from the surface and drying it followed by placing it on amorphous double sided tape and putting in a diffractometer. Angles from 20-80 degrees were scanned using 0.3 step size and 3 second dwell time. CuK_{α} source was used in this specific Siemens Krystalloflex Diffractometer.

The XRD spectrum of FIG. 5(a) shows the presence of tricalcium phosphate and monetite after a 2 hour 900 °C heat treatment. The XRD spectrum of FIG. 5(b) shows the presence of monetite and hydroxyapatite immediately following injection mineralization, but prior to any heat treatments.

### Example 10 - Slip casting of fiber matrix.

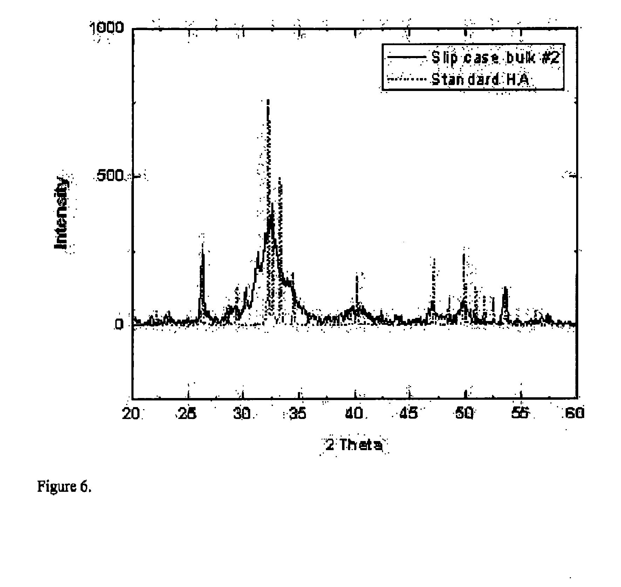

Demineralized bone matrix (10g) (Grafton Matrix, Osteotech, Inc., Eatontown, NJ) was soaked in 100mL of the calcium solution until hydrated (about 1 hour). Phosphate solutions were added as follows: 85mL of the unneutralized solution was added, followed by 15mL of the neutralized solution. All 4 components were then stirred until a thin white slurry results. The mixture was then poured onto a plaster of paris mold (slip casting mold) of desired shape and allowed to dry for about 48 hours, depending upon the thickness and shape of the mold. The mold was then placed in a 45 °C oven overnight to remove residual moisture. The XRD spectrum of FIG. 6 shows that hydroxyapatite is the dominant phase.

The foregoing examples and description of the preferred embodiments should be taken as illustrating, rather than as limiting the present invention as defined by the claims. As will be readily appreciated, numerous variations and combinations of the features set forth above can be utilized without departing from the present invention as set forth in the claims. Such variations are not regarded as a departure from the spirit and script of the invention, and all such variations are intended to be included within the scope of the following claims.

## Claims

1. A material comprising hydroxyapatite particles and a polymeric material, wherein the particles have a BET surface area between about 200 m²/g and about 3000 m²/g and a particle size between about 1 nm and about 9 nm.

2. A material comprising hydroxyapatite particles and a drug molecule, wherein the particles have a BET surface area between about 200 m²/g and about 3000 m²/g and a particle size between about 1 nm and about 9nm.

3. The material of claim 1 or claim 2, further comprising one or more dopant ions.

4. The material of claim 3, where the dopant ion is selected from the group consisting of ions of magnesium, fluorine, chlorine, potassium, Iron, carbonate, sodium, rare earth metals and combinations thereof.

5. The material of claim 1, further comprising a drug molecule.

6. The material of claim 1 or claim 2, further comprising a protein.

7. The material of claim 6, wherein the protein is selected from the group consisting of osteocalcin, osteonectin, bone morphogenetic proteins (BMPs), interleukins (ILs), glycoseminoglycans, proteoglycans, growth factors, fibrin, fibrinogen, chitosan, osteoinductive factor, fibronectin, human growth hormone, insulin-like growth factor, soft tissue, bone marrow, serum, blood, bioadhesives, human alpha thrombin, transforming growth factor beta, epidermal growth factor, platelet-derived growth factors, fibroglast growth factors, periodontal ligament chemotactic factor, somatotropin, bone digestors, antitumor agents, immune-suppressants, permeation enhancers, enamine derivatives, alpha-keto aldehydes, nucleic acids, amino acids and gelatin.

8. The material of claim 2 or claim 5 wherein the drug molecule is selected from the group consisting of osteocalcin, osteonectin, bone morphogenetic proteins (BMPs), interleukins (ILs), glycosaminoglycans, proteoglycans, growth factors, fibrin, fibrinogen, chitosan, osteoinductive factor, fibronectin, human growth hormone, insulin-like growth factor, soft tissue, bone marrow, serum, blood, bioadhesives, human alpha thrombin, transforming growth factor beta, epidermal growth factor, platelet-derived growth factors, fibroglast growth factors, periodontal ligament chemotactic factor, somatotropin, bone digestors, antitumor agents, immunosuppressants, permeation enhancers, enamine derivatives, alpha-keto aldehydes, nucleic acids, amino acids, gelatin, bisphosphonates, alendronate, strontium ranelate, teriparatide. Estradiol, Cypionate, Ethynyl Estradiol, Mestranol, Quinestrol, Exemestane, Testolactone, Norethindrone, Norethynodrel, Levonorgestrel, mifepristone, alkylating agents, antimetabolites, anthracyclines, alkaloids, topoisomerase inhibitors, monoclonal antibodies, tyrosine kinase inhibitors, antitumor antiblotics, paclitaxel, Cisplatin. Carboplatin, Oxaliplatin, Mechlorethamine, Chlorambucil. Cyclophosphamide, Ifosfamide, Busulfan, Camustine, Dacrbazine, Temozolomide, Procarbazine hydrochloride. Thiotepa, 5-Fluorouracil, Floxuridine, Capecitabine, Gemcitabine, Cytarabine, e-mercaptopurine, 6-thioguanine, Fludarabine phosphate, Cladribine, Clofarabine, Pentostatin, Methotrexate, paclitaxel, Docetaxel, Vincristine, Viblastine, Vinorelbine, camptothecin, Irinotecan, Topotecan, 5H-Dibenzo[c,h][1 ,6]-naphthyrindin-6-ones ARC-III, Etoposide, Doxorublein, Daunorubicin, Idarubicin, Novatrone, Bleomycin, Dactinomycin. Mitomycin, hydroxyurea, L-Asparaginase. Estramustine, Imatinib Mesylate, Dasatinib, Sorafenib, Sunitinib, Amifostine, MESNA, Dexrazoxane, Lucovorin Calcium, steroids, Tamoxifen citrate, Toremifene citrate, Enclomiphene citrate, Zuclomiphene, Anastrozole, Letrozole, antibiotics, pain relievers, analgesics, nutritional supplements, antihistamines, NSAIDS, antipsychotics, antichoiner-gics, cholinergics, antispasmotics, alpha and beta blockers, antidepressants, diabetes treatments, antivirals, dopaminergic agents, seratonergic agents, phosphodlesterase inhibitors, cardiac stimulants and suppressants, gastrointestinal drugs, antilipidemics, antihypertensive agents, diuretics, enzyme inhibitors, ion channel blockers, antifungal agents, steroids, blood glucose regulators, antiepileptics, anesthetics, skeletal muscle relaxants, prostaglandins, sedatives, analeptics, antineoplastics, antipro-tozoals, antihelminthics, hypnotics, antiemetics, antianginal, antiarrhythmics, vasodilators, vasocons-trictors, antiulcer agents, antiallergics, antacids, gene transfection agents, compositions for treating cancer and combinations thereof.

9. The material of claim 1, wherein the polymer is a biocompatible polymer selected from the group consisting of polysaccharides, poly(alkylene oxides), polyarylates, block copolymers of poly(alkylene oxides) with polycarbonates, polycarbonates, free acid polycarbonates, polyamide carbonates and polyester amides of hydroxy acids, polymers of L-tyrosine derived diphenol compounds, strictly alternating poly(alkylene oxide) ethers, polyacrylamide, polyacrylamide resin, modified poly(acrylic acid-co-hypophosphite), sodium salt polyacrylic acid, sodium salt poly(alkyl(C₁₆₋₂₂) acrylate), polydextrose, poly(divinylbenzene-co-ethylstyrene), poly(divinylbenzene-co-trimethyl(vinylbenzyl) ammonium chloride), polyethylene (m.w. 2,00-21,000), polyethylene glycol, polyethylene glycol (400) dioleate, polyethylene (oxidlzed), polyethyleneimine reaction product with 1,2-dichloroethane, polyglycerol esters of fatty acids, polyglyceryl phthalate ester of coconut oil fatty acids, polyisobutylene (min. m.w. 37,000), polylimonene, polymalelc acid, polymaleic acid, sodium salt, poly(maleic anhydride), sodium salt, polyoxyethylene dioleate, polyoxyethylene (600) dioleate, polyoxyethylene (600) mono-rici noleate, polyoxyethylene 40 monostearate, polypropylene glycol (mw. 1,200-3,000), polysorbate 20, polysorbate 60, polysorbate 65, polysorbate 80, polystyrene, cross-linked, chloromethylated, then aminated with trimethylamine, dimethylamine, diethylenetriamine, or triethanolamine, polyvinyl acetate, polyvinyl alcohol, polyvinyl polypyrrolidone, and polyvinylpyrrolidone polyamides, polyesters, polycaprolactone, polyglycolide-co-caprolactone, polyethylene oxide, polypropylene oxide, polyglycolide-cotrimethylene carbonate, poly(lactlc-co-glycollc acid), polylactide, polyglycolic acid, poly-L-lactide, polyethylene glycol, polypropylene, polyethylene and polyetheretherketones.

10. The material of claim 1 or claim 2, wherein the hydroxyapatite is stoichiometric.

11. The material of claim 1 or claim 2, wherein the hydroxyapatite is non-stoichiometric.

12. The material of claim 1 or claim 2, wherein the hydroxyapatite is amorphous.

13. The material of claim 1 or claim 2, further comprising a biomarker.

14. A drug delivery composition comprising the material of claim 1 or claim 2.

15. The drug delivery composition of claim 14 further comprising a drug molecule selected from the group consisting of osteocalcin, osteonectin, bone morphogenetic proteins (BMPs), interleukins (ILs), glyroseminoglycans, proteoglycans, growth factors, fibrin, fibrinogen, chitosan, osteoinductive factor, fibronectin, human growth hormone, Insulin-like growth factor, soft tissue, bone marrow, serum, blood, bioadhesives, human alpha thrombin, transforming growth factor beta, epidermal growth factor, platelet-derived growth factors, fibroblast growth factors, periodontal ligament chemotactic factor, somatotropin, bone digestors, antitumor agents, immune-suppressants, permeation enhancers, enamine derivatives, alpha-keto aldehydes, nucleic acids, amino acids, gelatin, bisphosphonates, alendronate, strontium ranelate, teriparatide, Estradiol Cypionate, Ethynyl Estradiol, Mestranol, Quinestrol, Exemestane, Testolactone, Norethindrone, Norethynodrel, Levonorgestrel, mifepristone, alkylating agents, antimetabolites, anthracyclines, alkaloids, topolsomerase inhibitors, monoclonal antibodies, tyrosine kinase inhibitors, antitumor antibiotics, paclitaxel, Cisplatin, Carboplatin. Oxaliplatin, Mechlorethamine, Chlorambucil, Cyclophosphamide, Ifosfamide, Busulfan, Camustine, Dacrbazine, Temozolomlde, Procarbazine hydrochloride, Thiotepa, 5-Fluorouracil, Floxuridine, Capecitabine. Gemcitabine, Cytarabine, 6-mercaptopurine, 6-thioguanine, Fludarabine phosphate, Cladribine, Clofarabine, Pentostatin, Methotrexate, paclitaxel, Docetaxel, Vincristine, Viblastine, Vinorelbine, camptothecin, Irinotecan, Topotecan, 5H-Dibenzo[c,h][1,6]naphthyrindin-6-ones ARC-III, Etoposide, Doxorubicin, Daunorubicin, Idarubicin, Novatrone, Bleomycin, Dactinomycin, Mitomycin, hydroxyurea, L-Asparaginase, Estramustine, Imatinib Mesylate, Dasatinib, Sorafenib, Sunitinib, Amifostine, MESNA. Dexrazoxane, Lucovorin Calcium, steroids, Tamoxifen citrate, Toremifene citrate, Enclomiphene citrate, Zuclomiphene, Anastrozole, Letrozole, antibiotics, pain relievers, analgesics, nutritional supplements, antihistamines, NSAIDS, antipsychotics, antichoinergics, cholinergics, antispasmotics, alpha and beta blockers, antidepressants, diabetes treatments, anti-virals, dopaminergic agents, seratonergic agents, phosphodiesterase inhibitors, cardiac stimulants and suppressants, gastrointestinal drugs, antilipidemics, antihypertensive agents, diuretics, enzyme inhibitors, ion channel blockers, antifungal agents, steroids, blood glucose regulators, antiepileptics, anesthetics, skeletal muscle relaxants, prostaglandins, sedatives, analeptics, antineoplastics, antiprotozoals, antihelminthics, hypnotics, antiemetics, antianginal, antiarrhythmics, vasodilators, vasoconstrictors, antiulcer agents, antiallergics, antacids, gene transfection agents, and compositions for treating cancer and combinations thereof.
